# EUROPÄISCHE PATENTSCHRIFT

(11) **EP 1 006 390 B1**
(45) Veröffentlichungstag und Bekanntmachung des Hinweises auf die Patenterteilung: **09.07.2003**
(21) Anmeldenummer: 98122900.8
(22) Anmeldetag: 02.12.1998
(51) Int. Cl.: G02B 21/00, G02B 23/24, A61B 1/00, G02B 7/00

(54) **Endomikroskopsystem**
Microscope/endoscope assembly
Ensemble microscope-endoscope

(43) Veröffentlichungstag der Anmeldung: 07.06.2000
(73) Patentinhaber: Möller-Wedel GmbH, 22880 Wedel (DE)
(72) Erfinder: Fenske, Christian Dr., 21335 Lüneburg (DE); Schmidt, Martin Dr., 23611 Bad Schwartau (DE); Draeger, Jörg Prof.Dr., 22299 Hamburg (DE)
(74) Vertreter: Glawe, Delfs, Moll & Partner

(56) Entgegenhaltungen:
- EP-A- 0 335 523
- DE-A- 4 422 522
- US-A- 4 862 873
- US-A- 5 295 477

## Beschreibung

Die Erfindung betrifft ein Endomikroskopsystem, bestehend aus einem Operationsmikroskop und einem flexiblen Bildübertragungssystem.

In verschiedenen medizinischen Disziplinen werden Operationsmikroskope eingesetzt, um mikrochirurgische Arbeiten an feinen Strukturen auszuführen. So gibt es spezielle Operationsmikroskope für die Ophthalmologie, Neurochirugie, Dentalmedizin und zahlreiche weitere Gebiete.

In manchen Fällen jedoch ist die direkte Sicht durch das Mikroskop begrenzt. So kann in der Ophthalmologie durch Blutungen die Sicht durch den Glaskörper auf die Netzhaut be- oder verhindert sein. In der Neurochirurgie besteht der Wunsch beim Zugang durch das Gehirn zu einem Tumor, diesen von der Seite oder Unterseite zu betrachten, um Verwachsungen mit Blutgefäßen oder Nerven frühzeitig zu erkennen. In der Dentalmedizin werden derzeit nur wenige Operationen überhaupt mit einem Operationsmikroskop ausgeführt, da die zerklüftete Topologie der Kiefer nur wenige Flächen von Zähnen oder Zahnfleisch erkennen lassen. Es ist weiterhin von Bedeutung, daß ein stereoskopisches Bild erzeugt wird, damit der Chirurg die Tiefe des Operationsfelds abschätzen kann.

Es sind Mikroskope bekannt, die durch eine kardanische Aufhängung eine besondere Beweglichkeit zulassen und Einsatz in der Neurochirurgie finden. Ein solches Mikroskop ist beschrieben in der DE-A-43 11 467. Das Problem, neben oder hinter eine Struktur zu blicken, kann jedoch auch durch dieses Mikroskop nicht gelöst werden. Auch kann dieses Mikroskop in der Dentalmedizin die Okklusion nicht wesentlich verbessern, da die Einblickrichtung von vornherein begrenzt ist.

In den Patentschriften DE-C-41 16 810, DE-C-42 25 507 sowie US-A-5,496,261 werden jeweils Kombinationen von Endoskopen mit Operationsmikroskopen bzw. operationsmikroskopähnlichen Einblicken an Endoskopen beschrieben. Auf diese Weise erreicht man zwar stereoskopisches Sehen durch ein Endoskop und kann ggf. das Endoskop wegschwenken, um das Oprationsmikroskop alleinig zu benutzten. Diese Kombinationen von Endoskopen und Operationsmikroskopen sind jedoch für den oben beschriebenen Einsatzzweck nicht geeignet. Die starre Kopplung von Mikroskop und Endoskop läßt keine freie Beweglichkeit der Endoskopspitze zu, so daß weiterhin Okklusionen auftreten. Zudem können Relativbewegungen zwischen Patienten, der in der Ophthalmonologie und Dentalmedizin jeweils nur örtlich betäubt ist, auftreten, wodurch die starre Anordnung nicht nur das Bild verlieren, sondern zu einer Gefahr für den Patienten werden kann.

In der DE-C-42 25 507 ist zwar die Verwendung von flexiblen Endoskopen beschrieben, durch die der letztgenannte Nachteil vermieden werden kann. Aus den Entgegenhaltungen ist aber zu entnehmen, daß durch das Endoskop ein Bild an einem Ort erzeugt wird, der dem Ort des Objektes entspricht, wenn das Mikroskop als gewöhnliches Mikroskop verwendet wird. Dabei befindet sich das Endoskop mit der Abbildungseinrichtung, mit der das zu betrachtende Bild erzeugt wird, auf der von der Objektivlinse entfernten Seite der Objektebene. Will man daher das vorher durch das Endoskop betrachtete Objekt direkt mit dem Mikroskop betrachten, so muß nicht nur das Endoskop entfernt werden, sondern auch das Mikroskop näher an das Objekt herangefahren und scharfgestellt werden. Dies bedeutet einen beträchtlichen Einstellaufwand.

Aus der DE 44 22 522 A1 ist ein Operationsmikroskop mit vorgeschaltetem schwenkaren, starren Endoskop bekannt, bei dem ein variabler Endoskopauszug durch ein Mikroskopobjektiv mit veränderlicher Schnittweite kompensiert wird. Die EP 0 335 523 A1 offenbart eine Vorrichtung mit optischen Elementen zum Verbinden eines Endoskops mit einem Kamerakopf.

Die Aufgabe der Erfindung besteht in der Schaffung eines Endomikroskopsystems, das so einstellbar ist, daß das Objekt ohne Neueinstellungen des Operationsmikroskops einerseits mit einem flexiblen Bildübertragungssystem und andererseits nach Entfernen desselben ohne dieses Bildübertragungssystem scharf abgebildet werden kann.

Die erfindungsgemäße Lösung besteht in einem Endomikroskopsystem nach Anspruch.

Das Abbildungssystem ist also nicht wie beim Stand der Technik von der Objektivlinse gesehen jenseits der Objektebene angeordnet, sondern zwischen Objektebene und Objektivlinse. Nach Entfernung des Bildübertragungssystems befindet sich dann die Objektebene deutlich unterhalb des Operationsmikroskops. Dabei kann die Anordnung ohne weiteres so gewählt werden, daß die Objektebene des Mikroskops mit der Objektebene des Endomikroskopsystems zusammenfällt, die aus dem Operationsmikroskop und dem flexiblen Bildübertragungssystem besteht. Man erhält also sowohl ein scharfes Bild durch das flexible Bildübertragungssystem als auch ohne Verstellung des Operationsmikroskops ohne dasselbe, wenn das Bildübertragungssystem aus dem Strahlengang entfernt ist.

Zweckmäßigerweise ist die Abbildungseinrichtung schwenkbar am Operationsmikroskop angebracht.

Das Endomikroskopsystem der Erfindung ist dabei so ausgebildet, daß die Abbildungseinrichtung optische Elemente enthält, die Bilder in oder nahe der Abbildungseinrichtung so in einen oder beide Strahlengänge des Operationsmikroskops abbilden, daß hinter der Objektivlinse desselben ein paralleles Strahlenbündel entsteht. Dieses kann dann wie bei einem normalen Mikroskop durch das Okular betrachtet werden.

Dabei ist vorgesehen, daß die optischen Elemente Sammellinsenanordnungen sind, deren Hauptebenen näher an den Bildern sind als deren Brennweite. Die Bilder werden also in der Art einer Lupe durch das Abbildungssystem abgebildet und dann vom Mikroskop betrachtet.

Bei einer vorteilhaften Ausführungsform ist vorgesehen, daß das Bildübertragungssystem zwei getrennte Übertragungskanäle besitzt, deren Bilder in der Abbildungseinrichtung getrennt in den beiden Strahlengängen des Operationsmikroskops eingestrahlt werden.

Wenn der Abstand der Enden der Übertragungskanäle auf der dem Objekt zugewandten Seite veränderbar ist (Stereovariator), so kann der Stereoeffekt vergrößert oder verkleinert werden. Bei einer vorteilhaften Ausführungsform besteht das Bildübertragungssystem aus einem oder mehreren flexiblen Endoskopen, insbesondere werden hier geordnete Faserbündel verwendet. Das Bild wird objektseitig auf das eine Ende des Faserbündels abgebildet. Das identische Bild am anderen Ende des Faserbündels wird dann über die Abbildungseinrichtung in den Mikroskopstrahlengang geleitet.

Bei einer anderen Ausführungsform besteht das Bildübertragungssystem aus einem oder mehreren Videoendoskopen. Für diesen Zweck sind CCD-Chips mit einem Durchmesser von lediglich ¼ Zoll (ca. 6 mm) bekannt, so daß diese an der Spitze des Bildübertragungssystems (Endoskops) angebracht werden können, das in den Körper eingeführt wird. Am anderen Ende des Bildübertragungssystems befinden sich dann Monitore, deren Bild genau wie das Bild des geordneten Faserbündels bei der ersten Ausführungsform durch die Abbildungseinrichtung in den Mikroskopstrahlengang geleitet wird.

Zweckmäßigerweise weist das Bildaufnahmesystem auf der dem Objekt zugewandten Seite einen Bildaufnehmer auf, in dem ein abbildendes optisches System angebracht ist, das durch ein Fenster von dem Objekt getrennt ist. Dabei ist es besonders zweckmäßig, wenn in den Bildaufnehmer eine Zufuhrleitung integriert ist, durch die Preßluft von einer externen Quelle leitbar ist, und das eine Auslaßöffnung für die Preßluft vorhanden und so gestaltet ist, daß die Preßluft über das Fenster strömen kann. Auf diese Weise kann das Fenster gereinigt werden.

Wie dies eingangs erwähnt wurde, soll die Abbildungseinrichtung aus dem Strahlengang entfernbar sein. Dies kann einerseits so geschehen, daß sie schwenkbar am Operationsmikroskop angebracht ist. Es ist aber auch möglich, daß das Bildübertragungssystem als an- und abmontierbares Modul ausgeführt ist.

Erfindungsgemäß kann vorgesehen sein, daß das Licht zur Beleuchtung des Beobachtungsfeldes durch das flexible Bildübertragungssystem geleitet wird. Das Bildübertragungssystem wird also nicht nur zum Betrachten des Beobachtungsfeldes verwendet, sondern auch zum Beleuchten. Es braucht also kein separater Lichtleiter oder dergleichen verwendet werden, so daß durch die Beleuchtung die in den Körper einzubringenden Elemente nicht vergrößert werden.

Bei einer vorteilhaften Ausführungsform ist eine separate Lichtquelle für das flexible Bildübertragungssystem vorgesehen, die zusammen mit dem Bildübertragungssystem vom Mikroskop entfernt werden kann, so daß die normale Betrachtung des Beobachtungsfeldes mit dem Mikroskop möglich ist. Bei einer anderen vorteilhaften Ausführungsform ist vorgesehen, daß das Licht der ohnehin vorhandenen Mikroskopbeleuchtung verwendet wird, so daß auf eine zusätzliche Lichtquelle verzichtet werden kann. Zweckmäßigerweise erfolgt die Einkupplung des Lichts in die Abbildungseinrichtung, die an dem schwenkbaren Operationsmikroskop angebracht ist, so daß hier keine weiteren Verbindungen für die Beleuchtung herzustellen oder zu trennen sind, wenn das flexible Bildübertragungssystem mit dem Mikroskop verbunden oder von demselben gelöst wird.

Die erfindungsgemäße Anordnung kann im wesentlichen beschrieben werden durch die Kombination eines Operationsmikroskops mit einem flexiblen Bildübertragungssystem, das entsprechend einem flexiblen Endoskop eingesetzt wird, dessen Bild aber in das Operationsmikroskop eingespiegelt wird. Wesentlich ist dabei, daß die Einspiegelung mittels eines optischen Systems dicht unterhalb der Objektivlinse des Operationsmikroskop erfolgt. Durch die Nähe zum Objektiv bleibt der Arbeitsabstand erhalten und das Objekt wird scharf durch das Operationsmikroskop gesehen, wenn die Einspiegelungsvorrichtung weggeschwenkt ist. Außerdem wird beim Einschwenken der Einspiegelungsvorrichtung der Sehweg zum Objekt verdeckt, so daß nur das endoskopische Bild gesehen wird.

Die Erfindung wird im folgenden anhand von vorteilhaften Ausführungsformen unter Bezugnahme auf die beigefügten Zeichnungen beschrieben. Es zeigen:
- Fig. 1: in schematischer Ansicht eine erste Ausführungsform;
- Fig. 2: in schematischer Ansicht eine zweite Ausführungsform der Erfindung; und
- Fig. 3: schematisch eine Einrichtung zur Veränderung des Abstands der Übertragungskanäle.

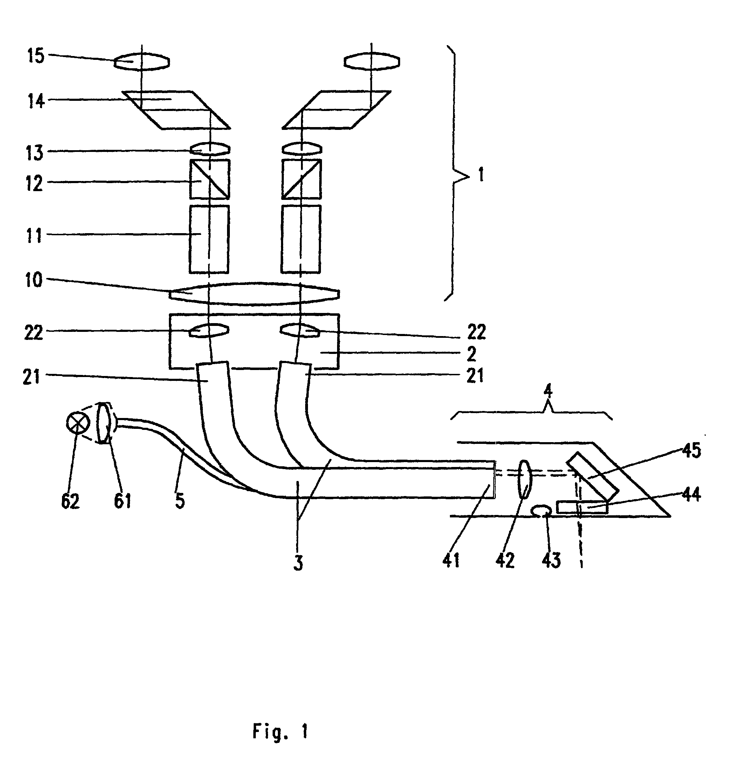

Figur 1 zeigt ein Operationsmikroskop 1, das aus einer Objektivlinse 10, einem Zoom-System 11, Strahlenteilern 12 zum Auskoppeln eines Teils des vom Objekt kommenden Lichts zu Kameras oder Mitbeobachtern und einer Abbildungsoptik 13 besteht, über der sich ein Umkehrsystem 14 und Okulare 15 befinden. Zwischen der Objektivlinse 10 und der Abbildungsoptik 13 besteht ein paralleler optischer Strahlengang.

Unterhalb der Objektivlinse 10 des Operationsmikroskops 1 befindet sich eine einschwenkbare Abbildungseinrichtung 2, in der sich optische Elemente 22 befinden, die das aus Lichtleiterenden 21 austretende Licht so abbilden, daß im Zusammenhang mit der Objektivlinse 10 ein paralleler Strahlengang entsteht. Dazu bestehen die optischen Elemente 22 aus Sammellinsen oder sind als Sammellinsen wirkende Linsensysteme, deren Abstand, von der Hauptebene aus gerechnet, zu den Lichtleiterenden 21 kleiner ist als deren Brennweite.

Die Lichtleiter 3, je einer für den linken und rechten Sehkanal, bestehen aus geordneten Faserbündeln. Eine Abbildung eines Objekts auf das distale Ende 41 eines Lichtleiters 3 wird weitergeleitet und erzeugt ein entsprechendes Bild am proximalen Ende 21, das dann letztlich durch die Okulare des Operationsmikroskops 1 scharf gesehen werden kann.

Auf der distalen Seite enden die Lichtleiter 3 in dem beweglichen Bildaufnehmer 4. Dieser besteht vorzugsweise aus einer Hülle mit einem Fenster 44, durch das das Licht von einem Objekt über einen Spiegel 45 mittels der Optik 42 auf die Lichtleiterenden 41 abgebildet wird. Nahe dem Fenster 44 ist eine Austrittsöffnung für Preßluft angebracht, damit eine Reinigung von Aerosolen erfolgen kann. Die Zuleitungen von einer Preßlufterzeugungseinheit bis zur Austrittsöffnung 43 ist im Beispiel der Übersichtlichkeit wegen nicht gezeigt.

Über einen weiteren Lichtleiter 5, der aus einem ungeordneten Faserbündel bestehen kann, wird Beleuchtungslicht zu dem Operationsfeld gelenkt. Das Beleuchtungslicht wird entweder in einer separaten Lichtquelle 62 erzeugt und über eine Beleuchtungsoptik 61 in den Lichtleiter 5 eingekoppelt oder dem Beleuchtungslicht des Operationsmikroskops (nicht gezeigt) entnommen. Im letzeren Fall sind der Lichtleiter 5 und die Einkoppeloptik 61 vorzugsweise in der Einspiegelungsvorrichtung 2 integriert, so daß nach deren Wegschwenken das Operationsmikroskop ohne Einschränkung funktionsfähig ist.

Die Beweglichkeit der Lichtleiter 3 ist von großer Bedeutung. Dadurch kann der Chirurg, wenn er mit dem beweglichen Bildaufnehmer 4 arbeitet, diesen in der Hand halten und gleichzeitig am Körper des Patienten abstützen. Dadurch führen kleinere Bewegungen des Patienten nicht zu Relativbewegungen zwischen Objekt und Bildaufnehmer 4.

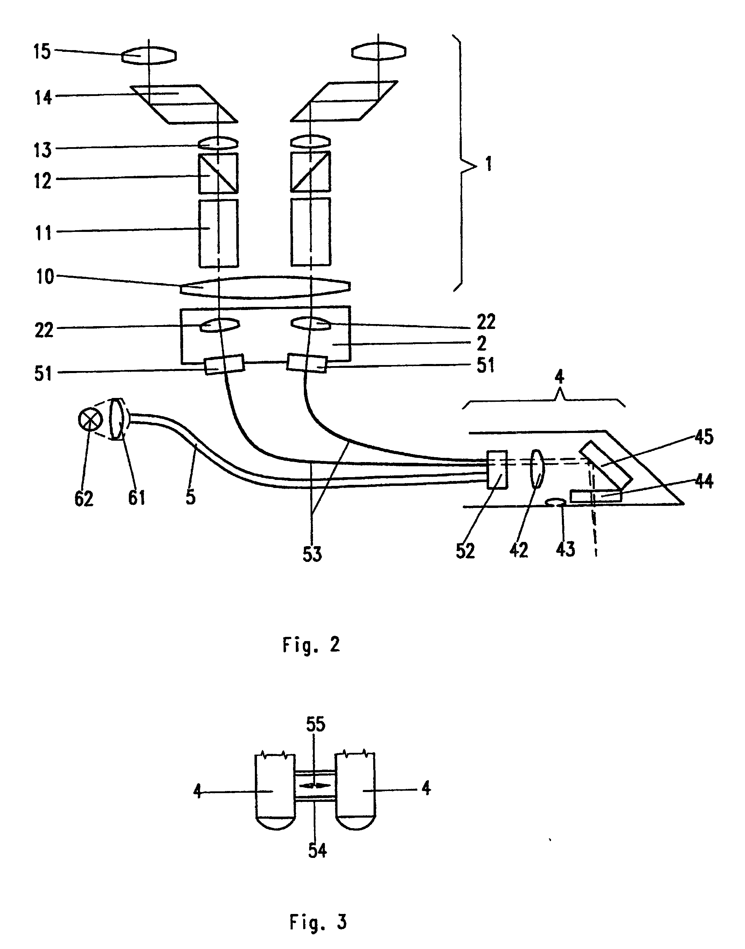

Bei der Ausführungsform der Figur 2 sind statt der geordneten Faserbündel Videokameras vorgesehen. Auf CCD-Chips 52 wird mit der gleichen optischen Abbildungseinrichtung 42,44,45 wie bei der Ausführungsform der Figur 1 jeweils ein Bild erzeugt, das dann mit Hilfe von Leitungen 53 zu Monitoren 51 übertragen wird, deren Bilder dann wie bei der Ausführungsform der Figur 1 in den Mikroskopstrahlengang geleitet werden. Die entsprechenden kleinen Monitore sind aus den Suchern von Videokameras bekannt. Die Lichtzufuhr zu dem Objekt kann wiederum durch eine kleine Lichtquelle im Bildaufnehmer 4 oder einen Lichtleiter 5 erfolgen, da das Licht von einer externen Lichtquelle oder, im eingeschwenkten Zustand, vom Beleuchtungslicht des Mikroskops erhält.

Der Bildaufnehmer 4 kann, der Aufgabe entsprechend, mit einer Vorrichtung vorsehen sein, die den Abstand von zwei getrennten Bildaufnehmern 4, wie dies in Figur 3 durch Führungen 54 und den Doppelpfeil 55 angedeutet ist, oder den Abstand der Lichtleiterenden 41 bzw. der Video-CCD-Chips 52 jeweils mit der zugehörigen Optik 42 variiert. Dadurch entsteht ein Stereovariator, der besonders vorteilhaft ist, wenn manchmal enge Kanäle eingesehen werden sollen auch unter teilweisem Verzicht auf den Stereoeffekt, der aber bei Oberflächenbetrachtungen maximiert werden soll.

Ebenfalls kann der Bildaufnehmer 4 auf medizinische Instrumente montiert werden, z. B. auf einen Zahnspiegel beim Einsatz in der Dentalchirurgie. Schließlich kann das ganze flexible Bildübertragungssystem als eigenständiges Modul ausgeführt sein, das getrennt vom Operationsmikroskop erworben und z. B. an ein bestehendes Operationsmikroskop anmontiert und bei Bedarf wieder entfernt werden kann.

## Patentansprüche

1. Endomikroskopsystem, bestehend aus einem Operationsmikroskop und einem flexiblen Bildübertragungssystem, das in einer Abbildungseinrichtung (2) endet, die zwischen der Objektivlinse (10) und der Objektebene des Operationsmikroskops (1) angeordnet ist und aus dem Strahlengang entfernbar ist,
wobei die Abbildungseinrichtung (2) optische Elemente (22) enthält, die Bilder in oder nahe der Abbildungseinrichtung (2) so in einen oder beide Strahlengänge des Operationsmikroskops (1) abbilden, daß hinter der Objektivlinse (10) ein paralleles Strahlenbündel entsteht, und
die optischen Elemente (22) Sammellinsenanordnungen sind, deren Hauptebenen näher an den Bildern sind als deren Brennweite, so daß die Objektebene des Operationsmikroskops bei entferntem Bildübertragungssystem mit der Objektebene des Endomikroskopsystems mit Bildübertragungssystem zusammenfällt.

2. Endomikroskopsystem nach Anspruch 1, **dadurch gekennzeichnet, daß** die Abbildungseinrichtung (2) schwenkbar am Operationsmikroskop (1) angebracht ist.

3. Endomikroskopsystem nach einem der vorangehenden Ansprüche, **dadurch gekennzeichnet, daß** das Bildübertragunssystem zwei getrennte Übertragungskanäle besitzt, deren Bilder in der Abbildungseinrichtung (2) getrennt in die beiden Strahlengänge des Operationsmikroskops (1) eingestrahlt werden.

4. Endomikroskopsystem nach Anspruch 3, **dadurch gekennzeichnet, daß** der Abstand der Enden (4,41,42,45) der Übertragungskanäle auf der dem Objekt zugewandten Seite veränderbar ist (Stereovariator).

5. Endomikroskopsystem nach einem der Ansprüche 1 bis 4, **dadurch gekennzeichnet, daß** das Bildübertragungssystem aus einem oder mehreren flexiblen Endoskopen besteht.

6. Endomikroskopsystem nach einem der Ansprüche 1 bis 5, **dadurch gekennzeichnet, daß** das Bildübertragungssystem aus einem oder mehreren Videoendoskopen, jeweils bestehend aus einer Videokamera (52), einer Signalübertragungseinheit (53) und einem Monitor (51) besteht.

7. Endomikroskopsystem nach einem der vorangehenden Ansprüche, **dadurch gekennzeichnet, daß** das Bildübertragungssystem auf der dem Objekt zugewandten Seite einen Bildaufnehmer (4) enthält, in dem ein abbildendes optisches System (42,44,45) angebracht ist, das durch ein Fenster (44) von dem Objekt getrennt ist.

8. Endomikroskopsystem nach Anspruch 7, **dadurch gekennzeichnet, daß** in den Bildaufnehmer eine Zufuhrleitung integriert ist, durch die Preßluft von einer externen Quelle leitbar ist, und daß eine Auslaßöffnung (43) für die Preßluft vorhanden und so gestaltet ist, daß die Preßluft über das Fenster (44) strömt.

9. Endomikroskopsystem nach einem der vorangehenden Ansprüche, **dadurch gekennzeichnet, daß** das Bildübertragungssystem als an- und abmontierbares Modul ausgeführt ist.

10. Endomikroskopsystem nach einem der vorangehenden Ansprüche, **dadurch gekennzeichnet, daß** das Licht zur Beleuchtung des Beobachtungsfeldes durch das flexible Bildübertragungssystem geleitet wird.

11. Endomikroskopsystem nach Anspruch 10, **dadurch gekennzeichnet, daß** es eine separate Lichtquelle (62) für das flexible Bildübertragungssystem aufweist.

12. Endomikroskopsystem nach Anspruch 10, **dadurch gekennzeichnet, daß** Licht der Mikroskopbeleuchtung verwendet wird.

## Claims

1. Endomicroscope system, comprising an operation microscope and a flexible image transmission system, which ends in an imaging device (2), which is arranged between the objective lens (10) and the object plane of the operation microscope (1) and can be removed from the beam path,
the imaging device (2) containing optical elements (22), which image images in or near the imaging device (2) into one or both beam paths of the operation microscope (1) in such a way that a parallel pencil of rays is produced behind the objective lens (10) and
the optical elements (22) being converging lens arrangements whose principal planes are nearer to the images than their focal length, so that the object plane of the operation microscope with the image transmission system removed coincides with the object plane of the endomicroscope system with the image transmission system.

2. Endomicroscope system according to Claim 1, **characterized in that** the imaging device (2) is fitted pivotably on the operation microscope (1).

3. Endomicroscope system according to one of the preceding claims, **characterized in that** the image transmission system has two separate transmission channels whose images, in the imaging device (2), are radiated separately into the two beam paths of the operation microscope (1).

4. Endomicroscope system according to Claim 3, **characterized in that** the spacing of the ends (4, 41, 42, 45) of the transmission channels on the side facing the object is variable (stereo variator).

5. Endomicroscope system according to one of Claims 1 to 4, **characterized in that** the image transmission system comprises one or more flexible endoscopes.

6. Endomicroscope system according to one of Claims 1 to 5, **characterized in that** the image transmission system comprises one or more video endoscopes, each comprising a video camera (52), a signal transmission unit (53) and a monitor (51).

7. Endomicroscope system according to one of the preceding claims, **characterized in that** the image transmission system on the side facing the object contains an image sensor (4), in which there is fitted an imaging optical system (42, 44, 45) separated from the object by a window (44).

8. Endomicroscope system according to Claim 7, **characterized in that** a supply line through which compressed air from an external source can be conducted is integrated in the image sensor, and **in that** an outlet opening (43) is present for the compressed air and is configured in such a way that the compressed air flows across the window (44).

9. Endomicroscope system according to one of the preceding claims, **characterized in that** the image transmission system is designed as a module that can be mounted and demounted.

10. Endomicroscope system according to one of the preceding claims, **characterized in that** the light for illuminating the observation field is guided by the flexible image transmission system.

11. Endomicroscope system according to Claim 10, **characterized in that** it has a separate light source (62) for the flexible image transmission system.

12. Endomicroscope system according to claim 10, **characterized in that** light from the microscope illumination is used.

## Revendications

1. Système à endomicroscope constitué d'un microscope chirurgical et d'un système flexible de transmission d'image, qui se termine dans un dispositif de reproduction (2), lequel est disposé entre la lentille d'objectif (10) et le plan objet du microscope chirurgical (1), et peut être éloigné du parcours des rayons,
le dispositif de reproduction (2) contenant des éléments optiques (22) qui reproduisent des images dans ou à proximité du dispositif de reproduction (2), dans l'un ou les deux parcours de rayons du microscope chirurgical (1), de manière que derrière la lentille d'objectif (10) il se forme un faisceau de rayons parallèles, et
les éléments optiques (22) sont des agencements de lentilles collectrices dont les plans principaux sont plus proches des images que leur distance focale, ce qui fait que le plan objet du microscope chirurgical coïncide avec le plan objet du système à endomicroscope avec système de transmission d'image, lorsque le système de transmission d'image est retiré.

2. Système à endomicroscope selon la revendication 1, **caractérisé en ce que** le dispositif de reproduction (2) est placé de manière à pouvoir pivoter sur le microscope chirurgical (1).

3. Système à endomicroscope selon l'une des revendications précédentes, **caractérisé en ce que** le système de transmission d'image possède deux canaux séparés de transmission dont les images sont envoyées séparément dans le dispositif de reproduction (2), dans les deux parcours des rayons du microscope chirurgical (1).

4. Système à endomicroscope selon la revendication 3, **caractérisé en ce que** la distance des extrémités (4, 41, 42, 45) des canaux de transmission est variable sur le côté tourné vers l'objet (variateur stéréo).

5. Système à endomicroscope selon l'une des revendications 1 à 4, **caractérisé en ce que** le système de transmission d'image est constitué d'un ou de plusieurs endoscopes flexibles.

6. Système à endomicroscope selon l'une des revendications 1 à 5, **caractérisé en ce que** le système de transmission d'image est constitué d'un ou de plusieurs endoscopes vidéo, constitués chacun d'une caméra vidéo (52), d'une unité de transmission de signaux (53) et d'un moniteur (51).

7. Système à endomicroscope selon l'une des revendications précédentes, **caractérisé en ce que** le système de transmission d'image contient, sur le côté tourné vers l'objet, un dispositif de prise de vues (4) dans lequel est placé un système optique (42, 44, 45) de reproduction, qui est séparé de l'objet par une fenêtre (44).

8. Système à endomicroscope selon la revendication 7, **caractérisé en ce que** dans le dispositif de prise de vues est intégrée une ligne d'alimentation par laquelle l'air comprimé est acheminé depuis une source externe, et **en ce qu'**une ouverture d'échappement (43) est prévue pour l'air comprimé et conçue de manière que l'air comprimé s'écoule à travers la fenêtre (44).

9. Système à endomicroscope selon l'une des revendications précédentes, **caractérisé en ce que** le système de transmission d'image est réalisé en tant que module pouvant être monté et démonté.

10. Système à endomicroscope selon l'une des revendications précédentes, **caractérisé en ce que** la lumière d'éclairage du champ d'observation est guidée par le système flexible de transmission d'image.

11. Système à endomicroscope selon la revendication 10, **caractérisé en ce qu'**il comporte une source de lumière (62) séparée pour le système flexible de transmission d'image.

12. Système à endomicroscope selon la revendication 10, **caractérisé en ce qu'**on utilise la lumière de l'éclairage du microscope.
